# DEMANDE DE BREVET EUROPEEN

(11) **EP 1 077 061 A2**
(43) Date de publication de la demande: **21.02.2001**
(21) Numéro de dépôt: 00402276.0
(22) Date de dépôt: 11.08.2000
(51) Int. Cl.: A61K 7/20, A61K 31/23, A61P 1/02

(54) **Utilisation de lipides peroxydés dans le traitement ou la prévention des plaies et inflammations des muqueuses de la cavité buccale**

(30) Priorité: 16.08.1999 FR 9910514
(71) Demandeur: Desjonqueres, Stéphane, 78600 Maisons-Laffitte (FR)
(72) Inventeur: Desjonqueres, Stéphane, 78600 Maisons-Laffitte (FR)
(74) Mandataire: Giraud, Françoise

(57) **Abrégé**

L'invention concerne l'utilisation de lipides peroxydés pour la fabrication d'une composition destinée au traitement ou à la prévention des plaies et inflammations superficielles des muqueuses de la cavité buccale, par formation d'un film protecteur sur lesdites muqueuses et/ou par effet de massage desdites muqueuses.

Elle s'applique tout particulièrement au traitement des aphtes, de la gingivite et de l'inflammation des gencives liées au port d'appareils dentaires.

Le taux de peroxydation est avantageusement compris entre 5 et 600 milliéquivalents par kilogramme.

## Description

La présente invention concerne une nouvelle utilisation des lipides peroxydés comme agents destinés à prévenir ou traiter les plaies ou inflammations des muqueuses de la cavité buccale.

Il est bien connu que la cicatrisation des plaies de la cavité buccale pose des problèmes particuliers liés à l'invasion salivaire et à l'agression de la mastication qui freinent le processus de cicatrisation spontanée de ces plaies.

Dans le cadre de ses recherches sur de nouveaux moyens de prévention et de traitement de ce type de plaie, l'inventeur de la présente invention a maintenant découvert que les lipides peroxydés pouvaient être utilisés de façon particulièrement efficace dans la prévention et le traitement de tous ces types de plaies de la cavité buccale, en apportant une solution originale consistant en une protection de type mécanique des lésions buccales, en particulier par formation d'un film protecteur et/ou par action de massage.

Ce type de mode d'action s'avère particulièrement intéressant, à une époque où l'on recherche particulièrement des modes de traitement aussi peu agressifs que possible pour l'organisme. Ils permettent, en outre, de satisfaire à la réglementation de la Communauté Européenne concernant les dispositifs médicaux, puisque ces derniers couvrent dans un chapitre particulier les produits permettant d'escompter des bénéfices sur la santé associés à des produits d'usage local par le seul effet mécanique lié par leur emploi.

On connaît différents lipides peroxydés, notamment obtenus par peroxydation d'huiles végétales naturelles. On citera, en particulier, les brevets suivants BSM N°2 330 M, EP-A-293 535, FR-A-2 591 112, EP-A-225 831, EP-A-225 832, EP-A-225 833, EP-A-226 506, FR-A-2 461 744, FR-A-2 539 142 et EP-A-117 962 qui concernent soit la préparation de tels lipides peroxydés soit leurs applications dans différents domaines, en particulier dans le traitement de certaines affections dans le domaine de la rhumatologie ou de la traumatologie, ou encore en tant que produit cicatrisant.

Il a maintenant été découvert que ces lipides peroxydés pouvaient être utilisés de façon particulièrement efficace dans le traitement ou la prévention des inflammations et des plaies superficielles des muqueuses de la cavité buccale, en mettant en oeuvre leur simple action mécanique, liée à la formation d'un film protecteur sur lesdites muqueuses ou à l'action de massage de ces muqueuses au moyen de compositions renfermant ces produits.

Ainsi, selon l'une de ses caractéristiques essentielles, l'invention concerne l'utilisation de lipides peroxydés présentant un taux de peroxydation compris entre 5 et 600 milli-équivalents par kilo, pour la fabrication d'une composition destinée au traitement ou à la prévention des plaies et inflammations superficielles des muqueuses de la cavité buccale, par formation d'un film protecteur sur lesdites muqueuses et/ou par effet de massage desdites muqueuses.

Les lipides peroxydés dont l'utilisation est revendiquée selon la présente invention résultent de la peroxydation de corps gras insaturés.

Le degré de peroxydation est mesuré selon la norme ISO 3960.

Pour la mise en oeuvre de la présente invention, on choisira des lipides peroxydés présentant un taux de peroxydation compris entre 5 et 600 milli-équivalents par kilo, de préférence entre 30 et 500 milli-équivalents par kilo.

Ce taux de peroxydation sera de façon encore plus avantageuse compris entre 50 et 300 milli-équivalents par kilo, de préférence encore entre 50 et 150 milli-équivalents par kilo.

Les lipides peroxydés préférés utilisés selon l'invention résultent de la peroxydation de lipides ou corps gras d'origine naturelle, de préférence d'origine végétale, de préférence encore de lipides issus d'une huile végétale naturelle.

A titre d'exemples d'huile naturelle choisie selon l'invention, on citera, l'huile d'amande douce, l'huile de noisette, l'huile d'arachide, l'huile de maïs, l'huile de pépin de raisin, l'huile de sésame et l'huile de carthame. On pourra également utiliser un mélange de ces huiles.

Selon une variante particulièrement préférée de l'invention, on choisira l'huile de maïs peroxydée et tout particulièrement une huile de maïs présentant un taux de peroxydation compris entre 5 et 600 milli-équivalents par kilo.

Les lipides peroxydés utilisés selon l'invention sont d'une façon avantageuse constitués, à titre de constituants majoritaires, représentant généralement au moins 80 % de la masse de triglycérides répondant à la formule 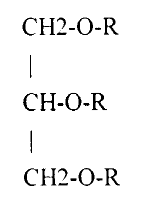 dans laquelle les radicaux R sont majoritairement représentés par des acides insaturés en C18 partiellement peroxydés (en fonction du taux de peroxydation dudit lipide).

Les compositions de l'invention peuvent être sous toute forme compatible avec une application topique à la surface des muqueuses de la cavité buccale. Ces compositions se trouveront de préférence sous la forme d'une émulsion de type huile-dans-eau ou eau-dans-huile, d'une crème, d'une pommade, d'un lait ou d'un gel, en particulier d'un gel huileux à base de silice colloïdale.

Toutefois, la forme préférée des compositions de l'invention est celle d'un gel huileux à base de silice colloïdale.

Parmi les affections de la cavité buccale pour le traitement et la prévention desquelles l'application d'une composition de l'invention s'avère particulièrement intéressante, on citera, en particulier, les aphtes, la gingivite, les manifestations douloureuses liées au port des appareils dentaires.

Plus précisément, les aphtes sont de petites ulcérations superficielles et douloureuses de la muqueuse buccale ou gingivale. Une telle affection évolue généralement sans complication vers la guérison. Toutefois, il est apparu que l'application d'une composition contenant une quantité efficace de lipides peroxydés permettait d'améliorer grandement la vitesse de cicatrisation de cette ulcération superficielle.

Dans le cas de ce traitement, la composition permet de former à la surface de l'aphte un film protecteur. L'application de ce film protecteur à la surface de la lésion de l'aphte va protéger cet abcès buccal des agressions extérieures, en particulier des agressions liées à la mastication de la nourriture et au mouvement de la langue, et, ce faisant, accélérer une évolution vers la guérison.

Ainsi, la protection par le film lipidique créé un environnement favorable à une accélération de la cicatrisation naturelle de l'aphte.

Dans le cas particulier des aphtes, on applique sur leur surface une composition contenant des lipides peroxydés. Cette composition agit en formant à la surface de l'aphte un film qui protège des sensations douloureuses de contact jusqu'à la guérison naturelle de l'aphte. Par ailleurs, on a pu montrer que l'application de lipides peroxydés participe à la restauration du film protecteur hydro-lipidique et accélère le renouvellement des cellules sous-jacentes en limitant la perte insensible en eau. De telles propriétés sont spécialement adaptées au soin des zones sensibles et n'impliquent aucun effet pharmacologique.

Ainsi, on améliorera considérablement la vitesse de cicatrisation des aphtes en appliquant simplement à leur surface une faible quantité de l'ordre d'un petit pois d'une composition contenant des lipides peroxydés, de préférence d'une composition sous forme de gel. Cette application pourra être faite simplement avec le doigt ou au moyen d'un coton tige et pourra être répété plusieurs fois par jour jusqu'à l'obtention d'une amélioration satisfaisante et en évitant de rincer la bouche immédiatement après l'application.

Comme il est exposé précédemment, dans ce type d'application on recourra de préférence à une composition sous forme de gel présentant une consistance adaptée pour pouvoir être aisément appliquée à la surface de l'aphte et y rester après application, de façon à protéger la lésion grâce à ses caractéristiques filmogènes.

Les compositions de l'invention contenant des lipides peroxydés conviennent également parfaitement pour le traitement ou la prévention de la gingivite.

La gingivite est une inflammation bien connue des gencives et peut être d'éthologies variées. En cas de durée prolongée ou d'intensité élevée, la gingivite nécessite généralement un avis, voire un suivi médical. Dans certains cas, la gencive fragilisée saigne légèrement lors du brossage des dents. Il est alors généralement reconnu qu'un massage gingival régulier des gencives peut prévenir ces saignements.

Or, il est apparu que les compositions de l'invention s'avèrent particulièrement efficaces pour le massage gingival par un effet purement mécanique. Par ailleurs, il a été montré que l'application des lipides peroxydés permet la restauration du film protecteur hydro-lipidique et accélère le renouvellement des cellules sous-jacentes en limitant la perte insensible en eau. Toutes ces propriétés s'avèrent spécialement adaptées au soin des zones sensibles sans être liées aucunement à un effet pharmacologique mais à un effet purement mécanique.

Dans le traitement de ces gingivites, comme dans celui des aphtes, il suffit de déposer un petit pois d'une composition contenant des lipides peroxydés à l'aide d'un coton tige ou du doigt, puis d'appliquer le produit sur la gencive et de masser doucement. Ce massage pourra être répété plusieurs fois par jour, en particulier après les repas, jusqu'à une amélioration satisfaisante et en évitant de rincer la bouche immédiatement après l'application.

Les compositions de l'invention contenant des lipides peroxydés s'avèrent également particulièrement intéressantes pour la prévention et le traitement des douleurs et inflammations liées au port des appareils dentaires. En effet, il est bien connu que le port des appareils dentaires peut être cause d'inflammation des gencives et de douleurs à ce niveau. Il est généralement reconnu qu'un massage gingival régulier agit sur la composante douloureuse de la sympthomatologie inflammatoire. Là encore, les compositions contenant des lipides peroxydés se sont avérées particulièrement bénéfiques en tant qu'agent de massage gingival régulier. Par ailleurs, là encore, des études ont montré que l'application des lipides peroxydés permettait de restaurer le film protecteur hydrolipidique et d'accélérer le renouvellement des cellules sous-jacentes en limitant la perte insensible en eau. Toutes ces propriétés sont liées à un effet purement mécanique et nullement pharmacologique.

Dans ce cas comme dans celui du traitement des gingivites, le mode d'application sera le même puisqu'il suffira d'appliquer sur les gencives une faible quantité, de l'ordre d'un petit pois d'une composition de l'invention, de préférence une composition sous forme de gel, au moyen d'un coton tige ou avec le doigt, puis de masser doucement les gencives en répétant le massage plusieurs fois par jour jusqu'à une amélioration satisfaisante et en évitant de rincer la bouche immédiatement après l'application.

Dans le traitement et la prévention des différentes affections de la cavité buccale données ci-dessus, on utilisera des compositions qui peuvent être sous la forme d'émulsion de type eau-dans-huile ou huile-dans-eau, de crème, de pommade, de lait ou de gel. Toutefois, une forme particulièrement avantageusement sera celle de gel huileux à base de silice colloïdale.

Les compositions utilisées contiendront généralement de 2 à 99 % en poids d'huile peroxydée. Ces compositions contiendront toutefois, de préférence, des quantités importantes d'huile peroxydée, de préférence entre 50 et 90 % en poids.

### EXEMPLES

Sauf indications contraires, les proportions données dans les exemples ci-dessous sont exprimées en pourcentage en poids.

### Exemple 1 : gel labial occlusif

- triesters de glycérol oxydés (T.G.O.) 90 %
- dioxyde de silicium sous forme de silice colloïdale 9 %
- huile de vaseline 0,5 %
- Parfum 0,4 %
- Aspartam 0,1 %

### Exemple 2 : gel buccal protecteur

- triesters de glycérol oxydés (T.G.O.) 92,70 %
- dioxyde de silicium sous forme de silice colloïdale 7 %
- saccharinate de sodium 0,20 %
- Arôme réglisse 0,10 %

### Exemple 3 : gel buccal destiné à la protection gingivale

- triesters de glycérol oxydés (T.G.O.) 92,70 %
- dioxyde de silicium sous forme de silice colloïdale 7 %
- saccharinate de sodium 0,20 %
- Arôme menthe 0,10 %

### Exemple 4 : gel destiné à la protection gingivale, en particulier dans le cas de port d'appareil dentaire

- triesters de glycérol oxydés (T.G.O.) 92,75 %
- dioxyde de silicium sous forme de silice colloïdale 7 %
- saccharinate de sodium 0,15 %
- Huile essentielle de clou de girofle 0,10 %

### Exemple 5 : mise en évidence de l'efficacité d'un gel protecteur contenant des lipides peroxydés sur les lésions buccales

L'objectif de l'étude réalisée dans cet exemple est de montrer clairement l'efficacité d'un gel répondant à la composition suivante :
- oxytriesters de glycérol de l'huile de maïs 90,5
- Aérosil® (silice colloïdale) 7
- excipient qsp
- arôme alimentaire menthe
dans le traitement des lésions buccales. On a réalisé une étude ouverte non comparative sur 137 patients. Cette étude a été réalisée en utilisant le gel dont la composition est donnée ci-dessus.

Les critères d'inclusion des patients ont été les suivants : gingivites, parodontopathies, inflammations post-implantaires, plaies prothétiques, aphtes.

Le protocole de réalisation du test est donné ci-dessous :

### Modalités d'applications

Une application sur la lésion après chaque repas et postérieurement à un brossage soigné des dents.

### Durée du traitement

8 jours.

### Critères de jugement

Evolution de la douleur, inflammation, gêne physiologique, rapidité d'action, durée d'action, amélioration du processus de guérison, évaluation globale du produit (agrément d'emploi, facilité d'application, tolérance, effet iatrogène, stabilité sur la lésion), cas particulier des aphtes, où l'on a comparé l'évolution de la crise avec celle des crises antérieures sans traitement selon l'invention.

Les résultats figurent dans le tableau I ci-dessous en ce qui concerne les aphtes et dans le tableau Il en ce qui concerne les autres lésions.

**TABLEAU I**

| Rapidité d'action en 2 heures ou moins | Durée d'action entre 2 et 4 heures | Amélioration en 2 jours ou moins |
|---|---|---|
| 66,7 % | 57 % | 55,6 % |

**TABLEAU II**

| Critère d'efficacité | Nombre de patients |
|---|---|
| Rapidité d'action en 2 heures | 70 % |
| Durée d'action entre 2 à 4 heures | 73,3 % |
| Amélioration de la symptomatologie en 2 jours ou moins | 62 % et 73,3 dans les plaies prothétiques |
| Agrément positif du produit | 65,1 % |
| Facilité d'application | 83,3 % |
| Tolérance locale excellente | 90 % |
| Bonne stabilité du produit sur la lésion | 79,1 % |

### Conclusion

Ces résultats illustrent l'intérêt de la protection des lésions superficielles buccales par une préparation à base d'huiles peroxydées.

### Exemple 6 : mise en évidence de l'efficacité des lipides peroxydés sur la cicatrisation de la gencive et de la diminution de l'oedème après blessures provoquées

Une étude en double aveugle a été réalisée pour comparer l'effet d'un produit cicatrisant ci-après désigné A avec un produit B en tous points identiques A mais dans lequel l'excipient a été remplacé par un excipient à base d'huile peroxydée.
a) Composition des produits testés
   - Produit A :
      Menthol en solution : 0,04 %
      Chlorure de cétylpyridinium à 0,1 % : 0,045 %
      Teinture de benjoïn 3 %
      Parfums
      Excipient aqueux qsp 100
   - Produit B :
      Menthol en solution : 0,04 %
      Chlorure de cétylpyridinium 0,045 %
      Teinture de benjoïn 3 %
      Parfums
      Lipides peroxydés 90 %
      Excipient aqueux qsp 100
b) Protocole du test
   Après une première visite de présélection, les individus retenus pour le test sont soumis à une deuxième visite au jour 0 (J 0). Au cours de cette visite, après anesthésie locale, on provoque une blessure au laser et le degré maximum de douleurs enregistrées est noté de 0 (aucune douleur) à 10 (douleur sévère). Les sujets sont ensuite répartis en 3 groupes soumis respectivement à l'un des traitements suivants :
   - traitement par le produit A,
   - traitement par le produit B,
   - pas de traitement.

   Les sujets ensuite appliquent suivant le cas avec le doigt une petite quantité de produit A, de produit B ou simplement miment cette action pour les sujets qui ne sont soumis à aucune application et notent l'intensité de la douleur au bout de 1 min, 10 min, 30 min et 50 min après l'application du produit. Les deux groupes qui appliquent le produit A ou B notent également au bout de 50 min l'adhérence du produit.
   Une nouvelle application de produit est ensuite effectuée au bout de 50 min et les niveaux de douleurs sont notés au bout de 1 min, 10 min, 30 min et 50 min après la deuxième application.
   Les sujets appliquent ensuite pendant les quatre premiers jours d'études deux fois par jour, suivant le cas soit le produit A, soit le produit B, soit un simple massage puis ils font trois applications pendant les derniers jours de l'étude.
   Les sujets notent à chaque application 5 min après l'application (ou le simulacre d'application) le degré de douleur et sont par ailleurs soumis quotidiennement à une visite médicale au cours de laquelle le médecin note le degré d'érythème, d'oedème ou l'apparence de la cicatrisation.
c) Résultats
   Le test clinique a été conduit pendant 5 jours sur une population de 60 sujets (20 sujets dans chacun des trois groupes testés) âgés de 18 à 65 ans en bonne santé.
   Les résultats ont clairement montré une meilleure fermeture des lèvres de la plaie en faveur du produit B et une activité démontrée sur les signes associés, en faveur du produit B.

## Revendications

1. Utilisation de lipides peroxydés présentant un taux de peroxydation compris entre 5 et 600 milli-équivalents par kilo, pour la fabrication d'une composition destinée au traitement ou à la prévention des plaies et inflammations superficielles des muqueuses de la cavité buccale, par formation d'un film protecteur sur lesdites muqueuses et/ou par effet de massage desdites muqueuses.

2. Utilisation selon la revendication 1, caractérisée en ce que ladite composition est destinée au traitement des aphtes, en particulier par formation d'un film protecteur.

3. Utilisation selon la revendication 1, caractérisée en ce que ladite composition est destinée au traitement et/ou à la prévention de la gingivite, en particulier par effet mécanique de massage des gencives.

4. Utilisation selon la revendication 1, caractérisée en ce que ladite composition est destinée au massage des gencives destinée à éviter l'inflammation des gencives liée au port d'appareils dentaires.

5. Utilisation selon l'une des revendications 1 à 4, caractérisée en ce que lesdits lipides peroxydés présentent un taux de peroxydation compris entre 30 et 500, de préférence encore entre 50 et 300, de préférence encore entre 50 et 150 milli-équivalents par kilo.

6. Utilisation selon l'une des revendications 1 à 5, caractérisée en ce que lesdits lipides peroxydés comprennent, à titre de constituants majoritaires, des triglycérides partiellement oxydés répondant à la formule générale : 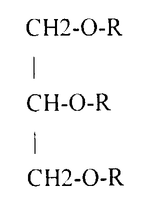 dans laquelle les radicaux R sont des acides insaturés en C18 partiellement peroxydés.

7. Utilisation selon l'une des revendications 1 à 6, caractérisée en ce que lesdits lipides peroxydés sont obtenus par peroxydation de lipides ou de corps gras d'origine naturelle, de préférence de lipides issus d'une huile végétale naturelle.

8. Utilisation selon la revendication 7, caractérisée en ce que l'huile naturelle est choisie dans le groupe constitué de l'huile d'amande douce, de l'huile de noisette, de l'huile d'arachide, de l'huile de maïs, de l'huile de pépin de raisin, de l'huile de sésame et de l'huile de carthame et de leurs mélanges.

9. Utilisation selon l'une des revendications 1 à 8, caractérisée en ce que ladite composition est sous la forme d'une émulsion de type huile-dans-eau ou eau-dans-huile, d'une crème, d'une pommade, d'un lait ou d'un gel, en particulier d'un gel huileux à base de silice colloïdale.

10. Utilisation selon la revendication 9, caractérisée en ce que ladite composition est sous la forme d'un gel huileux à base de silice colloïdale.

11. Utilisation selon l'une des revendications 1 à 10, caractérisée en ce que ladite composition contient de 2 à 99 % en poids, de préférence de 50 à 90 %, d'huile peroxydée.
